# EUROPEAN PATENT SPECIFICATION

(11) **EP 0 367 869 B1**
(45) Date of publication and mention of the grant of the patent: **07.04.1993**
(21) Application number: 88310440.8
(22) Date of filing: 07.11.1988
(51) Int. Cl.: C07C 37/62, C07C 39/367, B01F 5/04

(54) **Tetrabromobisphenol-A process**
Tetrabrombisphenol-A-Herstellungsverfahren
Préparation de tétrabromobisphénol-A

(43) Date of publication of application: 16.05.1990
(73) Proprietor: ETHYL CORPORATION, Baton Rouge Louisiana 70801 (US)
(72) Inventor: Mitchell, Olan Wayne, Magnolia Arkansas 71753 (US); McKinnie, Bonnie Gary, Baton Rouge Louisiana 70814 (US)
(74) Representative: Collier, Jeremy Austin Grey

(56) References cited:
- DE-A- 2 046 254
- US-A- 3 154 103
- US-A- 4 628 124

## Description

Tetrabromobisphenol-A (hereinafter "TBBP-A") is 4,4'-isopropylidenebis (2,6-dibromophenol). It is a widely used commercial fire retardant. There have been numerous publications on how it can be made. Hennis, U.S. 3,234,289, describes a process in which bisphenol-A (i.e., 4,4'-isopropylidenebisphenol) is placed in a water-alcohol mixture and liquid bromine is added at 22-28°C followed by reflux. Majewski et al., U.S. 3,363,007, discloses a process for brominating bisphenol-A in a mixture of water and an alkyl ether of a lower glycol.

Asadorian et al., U.S. 3,546,302, discloses a bromination process conducted in a two-phase solvent having an aqueous phase and an organic phase.

Montanari et al., U.S. 3,868,423, discloses the bromination of isopropylidenebisphenol with liquid bromine and gaseous chlorine in a methanol solvent. Janzon et al., U.S. 3,929,907, discloses the bromination of bisphenols in the presence of aqueous hydrogen peroxide.

Brackenridge, U.S. 4,013,728, teaches a process for brominating bisphenol-A in aqueous acetic acid followed by a heating step. Jenkner, U.S. 4,036,894, discloses bromination of bisphenol-A in acetic acid with recycle of the mother liquor and addition of alkaline or alkaline earth metal acetate.

Production of TBBP-A by dissolving bisphenol-A in methanol and adding liquid bromine is an effective way to make TBBP-A but the product contains various impurities which detract from its commercial value. These impurities include brominated phenols and hydrolyzable impurities. A need exists for a process that would lower the amount of these impurities.

It has been discovered that the amount of impurities in TBBP-A can be sharply decreased by using a process in which bisphenol-A is dissolved in methanol and brominated by adding a solution of bromine in methanol to the bisphenol-A methanol solution. It has been shown that the amount of impurities can be sharply reduced from about 4 weight percent down to as little as 0.2 weight percent. It has now been discovered that adaptation of this process into large scale equipment can be facilitated by pre-mixing the bromine-methanol feed and impinging this feed stream with a stream of the reaction mixture being circulated outside the reaction vessel through a closed loop.

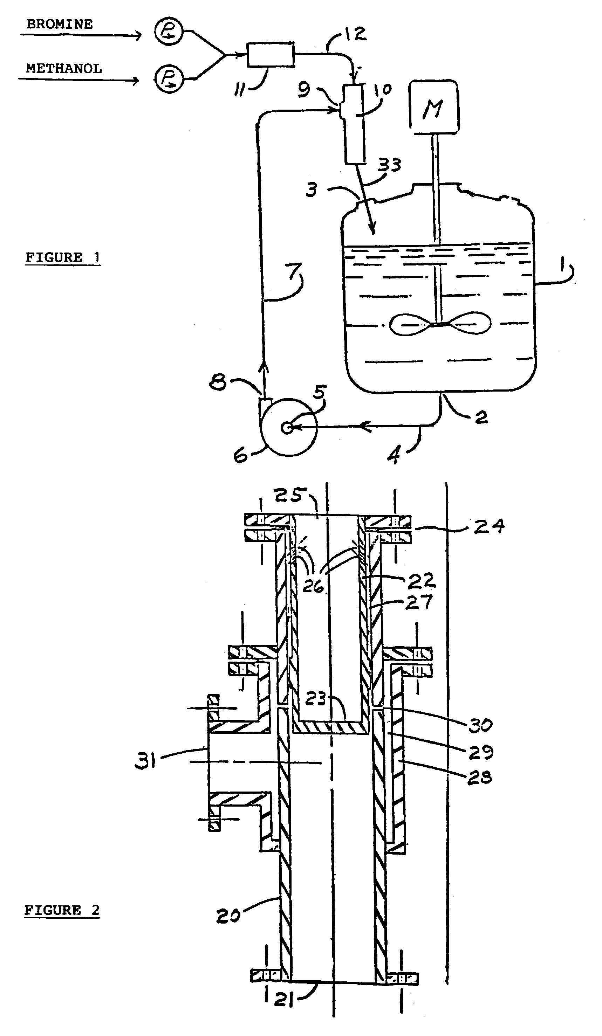

Figure 1 is a schematic representation of an embodiment of the process showing the reaction vessel and the external closed loop flow path through an impingement mixer.

Figure 2 is a cross-section of a suitable impingement mixer in which pre-mixed bromine-methanol feed is impinged with circulating reaction mixture in an annular space.

A preferred embodiment of the invention is a process for making TBBP-A in high yield and high purity, said process comprising:
(A) dissolving bisphenol-A in methanol in a reaction vessel,
(B) withdrawing a stream of the bisphenol-A/methanol solution from said reaction vessel and feeding this withdrawn stream to an impingement mixer,
(C) feeding a mixture of bromine and methanol to said impingement mixer,
(D) impinging said stream of bisphenol-A/methanol solution with said mixture of bromine and methanol in said impingement mixture forming a bromination mixture and
(E) conducting said bromination mixture back into said reaction vessel,
(F) continuing steps (B), (C), (D) and (E) until the desired amount of bromine has been fed and
(G) recovering tetrabromobisphenol-A.

Initially a reaction vessel 1, preferably glass or glass-lined, is charged with methanol and bisphenol-A. The amount of methanol used to dissolve the bisphenol-A can vary over a wide range. A useful range is 1.0-6 parts by weight methanol per each part bisphenol-A. A more preferred range is 1.5-3 parts by weight methanol per each part bisphenol-A and the most preferred amount is about 2.0 parts methanol per each part bisphenol-A.

The process is then started by activating pump 6 which withdraws reaction mixture (initially methanol-bisphenol-A solution) from bottom outlet 2 through outlet conduit 4. Pump 6 is preferably of the centrifugal type and receives the reaction mixture at suction intake 5. The reaction mixture is pumped through discharge port 8 and riser conduit 7 to impingement mixer 10 which will be described in detail later.

Concurrently bromine and methanol are pumped at a controlled rate from storage through conventional static mixer 11.

The bromine/alcohol ratio can vary widely. The more dilute the bromine solution, the better the results. However, excessive dilution causes an unacceptable drop in production per unit volume of reactor. A useful range in which to operate is 1-4 parts by weight bromine per each part methanol. A more preferred range is 1-3 parts bromine per each part methanol. The most preferred amount is about 2 parts bromine per each part methanol.

The ratio of (1) the volume of the reaction mixture recirculation through the external loop and impingement mixer to (2) the volume of the methanol-bromine solution feed can vary over a wide range. Preferably the volume of the reaction mixture recirculation will exceed the volume of the methanol-bromine feed. A useful range is 0.8-30:1. A more useful range is 10-20:1 and a most preferred range is 15-18:1.

The amount of methanol-bromine solution should be an amount that supplies sufficient bromine to make an acceptable product. The stoichiometric requirement is 4 moles of bromine per mole of bisphenol-A. A useful range in which to operate is 3.9-4.5 moles of bromine per mole of bisphenol-A and the most preferred range is 4.0-4.1 moles bromine per mole of bisphenol-A.

From static mixer 11 the bromine-methanol solution passes through conduit 12 to impingement mixer 10.

Impingement mixer 10 comprises an outer substantially cylindrical shell 20 open at its discharge end 21. Hollow distribution member 22, essentially closed at one end 23, is axially located inside shell 20 and sealably engaged with shell 20 at end 24 opposite discharge end 21. Distribution member 22 has an inlet 25 and a plurality of orifices 26 circumferentially located in the sidewall of member 22 forming a plurality of passages from the hollow interior of member 22 into annular space 27 between member 22 and shell 20.

Outer axial cylindrical member 28 is sealably engaged at both ends to shell 20 forming outer annular chamber 29. A circumferential slit 30 extends around shell 20 forming a narrow circumferential passage from annular chamber 29 into annular space 27. Side outlet 31 in member 28 is adapted to connect to riser conduit 7 to receive the reaction mixture.

The bromine-methanol solution from static mixer 11 passes through conduit 12 to inlet 25 of distribution member 22. The bromine-methanol solution is forced at high velocity through orifice 26 into annular space 27. Meanwhile reaction mixture from riser conduit 7 enters annular chamber 29 through side inlet 31 and is forced at high velocity through slit 30 into annular space 27.

The bromine-methanol solution and the reaction mixture impinge in annular space 27 and the resultant mixture passes through discharge end 21 which is operably connected to feed conduit 33 which feeds the mixture back into reactor 1.

The improvements of the present process enable the production of TBBP-A on a large scale at high yield to give a product that is substantially lower in impurities compared to TBBP-A made feeding bromine to a methanol-bisphenol-A-solution.

Rapid mixing of the bromine-methanol and bisphenol-A-methanol is highly preferred in order to obtain the best results with the new mode of bromine addition.

The bromine-methanol solution may be fed to the circulating reaction mixture at an initial temperature that is ambient or lower although this is not essential. For example the bromine-methanol feed can be started while the reactor contents and circulating reaction mixture are at temperatures from -10° up to 30° or somewhat higher if that is what the liquid happens to be at. As the feed progresses the temperature will rise due to the heat of the reaction. Sometime during the feed the reactor temperature will attain reflux conditions and reflux can be continued through the end of the feed of the bromine-methanol solution, although reflux is not essential as long as the reaction is continued long enough to substantially complete the bromination. After this, heat can be applied to maintain reflux for a short period of time of say 10 minutes to 1 hour to assure completion of the reaction.

During the bromine-methanol feed, the bromination of bisphenol-A forms HBr which reacts with the methanol to form methyl bromide. The methyl bromide vaporizes and can be collected from the off-gas and marketed as a commercial product for its many known uses such as soil fumigation.

TBBP-A can be recovered from the reaction mixture using conventional methods. For example the final reaction mixture can be diluted with water and filtered to recover the TBBP-A. The product can then be dried in an oven to remove water, methanol, bromine, HBr and other volatiles.

The following examples serve to illustrate how the process is carried out and to compare it to a prior art process using liquid bromine feed rather than feeding a bromine-methanol solution.

### EXAMPLE 1

### Comparative Example

In a reaction vessel fitted with a condenser, heating mantle, thermometer, stirrer and addition funnel with a dip tube was placed 223 grams of methanol (3% water) and 52.65 grams bisphenol-A. While stirring, this was heated to reflux and 154.5 grams of bromine was added through the dip leg over an 80 minute period at reflux. Reflux was continued for 8 minutes and then Na₂SO₃ was added to destroy unreacted bromine. A small sample of the product was removed and dissolved in methylene chloride, washed with water and dried over anhydrous sodium sulfate. The methylene chloride was evaporated and N,O-bis(trimethylsilyl)trifluoroacetamide added to derivatize the product which was then analyzed by gas chromatograph.

### EXAMPLE 2

### Comparative Example

This example is according to US-A-4628124. It was conducted using a feed of bromine-methanol to a methanol-bisphenol-A solution but without the use of an impingement mixer in an exterior circulating closed loop.

A reaction vessel was charged with 54.16 grams bisphenol-A and 122 grams methanol (3% water). A solution of 165 grams bromine in 85 grams methanol was prepared with cooling. While stirring at the same rate as in Example 1, the bromine-methanol solution was added slowly to the bisphenol-A solution starting at room temperature. When one-third of the bromine solution was added, the reaction mixture reached reflux. It was maintained at reflux through the remainder of the feed. Feed time was 84 minutes. Reflux was continued for 8 minutes. Sodium sulfite was added to destroy unreacted bromine. A sample of product was worked-up and derivatized as in Example 1 and analyzed by gas chromatography.

Analysis of the TBBP-A from Examples 1 and 2 is shown in the following table.

| Compound | Amount (area %) | |
|---|---|---|
| | Example 1 | Example 2 |
| TBBP-A | 95.57 | 99.14 |
| Tribromobisphenol-A | 0.05 | 0.277 |
| Dibromobisphenol-A | ND⁴ | 0.030 |
| Tribromophenol | 2.5 | 0.188 |
| Dibromophenol | ND | 0.068 |
| Bromophenol | ND | 0.014 |
| Compound A¹ | 0.83 | 0.078 |
| Compound B² | 0.22 | 0.036 |
| Compound C³ | 0.58 | 0.118 |
| Unknown | ND | 0.009 |

| | | |
|---|---|---|
| ¹ 1-bromo-2-(3,5-dibromo-4-hydroxyphenyl)-2-methoxypropane. | | |
| ² 1,1-dibromo-2-(3,5-dibromo-4-hydroxyphenyl)-2-methoxypropane. | | |
| ³ 1,3-dibromo-2-(3,5-dibromo-4-hydroxyphenyl)-2-methoxypropane. | | |
| ⁴ ND means present but not determined. | | |

The results show that the tetrabromobisphenol-A made in Example 2 is significantly higher in purity compared to Example 1 made by a prior art process.

The following example shows the use of a bromine-methanol feed as in Example 2 but includes the further improvement of this invention whereby the reaction mixture is circulated through an external closed loop and the bromine-methanol solution is injected into the closed loop in an impingement type mixer.

### EXAMPLE 3

In a glass lined reaction vessel was placed 1016 kg of methanol and 454 kg of bisphenol-A. When dissolved the solution was circulated through an external loop which included an impingement mixer at the rate of 946 liters per minute. A bromine-methanol solution (2:1 bromine: methanol weight ratio) was pumped to the impingement mixer at a rate of 943 liters/min. The bromine solution feed was continued until 1% stoichiometric excess over that required for TTBP-A had been fed. The temperature in the reaction vessel during bromine-methanol feed rose from 22°C to reflux over 20 min. Reflux was maintained until the bromine-methanol feed was complete. Following feed completion, the recirculation of reaction mixture was stopped and reflux was continued for 30 minutes. The reaction mixture was analyzed as in Example 1 with the results shown in the table below. Product was recovered by quenching with water and filtering.

| Compound | Example 3 | Typical¹ |
|---|---|---|
| TBBP-A | 98.03% | 91-96 |
| Tribromobisphenol-A | 0.09 | 0.5 |
| Tribromophenol | 0.60 | 2.5 |
| Dibromophenol | 0.06 | ND |
| Compound A | 0.24 | |
| Compound B | 0.14 | 1.6 |
| Compound C | 0.30 | |

| | | |
|---|---|---|
| ¹ Typical analysis of TBBP-A made on the same commercial scale but direct liquid bromine feed to the reactor. | | |

The results show that the improved process achieves a very high purity TBBP-A on a commercial scale.

The improved process is applicable to the bromination of other bisphenols. These are compounds of the structure: 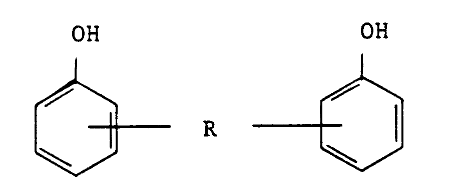
wherein R is a divalent aliphatic hydrocarbon group of 1-4 carbon atoms or a direct bond between the two benzene rings. Representative examples are 4,4'-methylenebisphenol, 2,2'-methylenebisphenol, 2,4'-methylenebisphenol, 4,4'-ethylidenebisphenol, 2,2'-ethylidenebisphenol, 2,4'-ethylidenebisphenol, 2,2'-isopropylidenebisphenol, 2,4'-isopropylidenebisphenol, 4,4'-butylidenebisphenol, 2,2'-butylidenebisphenol, 4,4'-biphenol, 2,2'-biphenol and 2,4'-biphenol. These bisphenols can be substituted for the bisphenol-A, i.e. 4,4'-isopropylidenebisphenol, used in the foregoing description and examples of the present invention. All of the products can be used as fire retardants in a broad range of organic materials normally susceptible to combustion in the presence of air and an ignition source.

## Claims

1. A process for making a brominated bisphenol in high yield and high purity, said process comprising:
(A) feeding a first stream comprising bromine dissolved in methanol to an impingement mixer;
(B) impinging a second stream comprising the bisphenol dissolved in methanol and, after at least one cycle of said process, brominated bisphenol with said first stream in said impingement mixer to rapidly mix said streams and to form a mixture thereof;
(C) conducting said mixture to a reaction vessel which holds reactor contents which comprise the bisphenol dissolved in methanol and, after at least one cycle of said process, the brominated bisphenol;
(D) removing a portion of said reactor contents from said reaction vessel to form said second stream; and
(E) continuing steps (A), (B), (C) and (D) until the desired amount of bromine has been fed.

2. The process of claim 1 wherein the bisphenol is bisphenol-A and the brominated bisphenol is tetrabromobisphenol-A.

3. The process of claim 2 wherein, at process initiation, the weight ratio of said methanol to said bisphenol-A in said second stream is 1-6:1 and the weight ratio of said bromine and methanol in said first stream is 1-3:1.

4. The process of Claim 3 wherein, at process initiation, the weight ratio of said methanol to said bisphenol-A in said second stream is 1.5-3:1 and the amount of bromine fed is 4.0-4.1 moles of bromine per mole of bisphenol-A used in said process.

5. The process of Claim 4 wherein the volume ratio of said second stream to said first stream is within the range of 10-20:1.

6. The process of any one of Claims 1 to 5 wherein the temperature in said reaction vessel attains reflux conditions during the course of the reaction.

7. The process of any one of Claims 1 to 6 wherein the brominated bisphenol is recovered from said process.

8. The process of Claim 7 wherein said recovery is effected by the steps comprising quenching the reactor contents with water to precipitate the brominated bisphenol and filtering said precipitated brominated bisphenol from said reactor contents.

9. An apparatus adapted to make an organobromine compound in high yield and high purity, said apparatus comprising a reaction vessel having a bottom outlet and an inlet, an outlet conduit forming a flow path from said bottom outlet to the intake of a pump, a riser conduit forming a flow path from the discharge port of said pump to the side inlet of an annular impingement mixer, a bromine source and a solvent source operatively connected by a bromine-solvent conduit to the end inlet of said annular impingement mixer, said annular impingement mixer comprising an outer substantially cylindrical shell, a substantially cylindrical hollow distribution member essentially closed at one end and axially located inside of and spaced apart from said cylindrical shell and sealably engaged with said shell at one end of said shell forming an annular space between said distribution member and said shell, openings in the sidewall of said distribution member forming a flow path from inside said distribution member into said annular space, an end inlet in said distribution member opposite said closed end, said end inlet being operatively connected to said bromine-solvent conduit, an outer axial cylindrical member around and spaced apart from said cylindrical shell, both ends of said axial cylindrical member being sealably connected to said cylindrical shell forming an annular chamber, fluid passage means around the circumference of said cylindrical shell forming a passage from said annular chamber into said annular space, an inlet in said cylindrical member functioning as said side inlet of said annular impingement mixer, a feed conduit forming a flow path from an outlet in said cylindrical shell remote from said distribution member to said inlet of said reaction vessel.

10. An apparatus of Claim 9 wherein said solvent source is a methanol source.

11. An apparatus of Claim 10 further characterized by having static mixing means in said bromine-solvent conduit.

## Patentansprüche

1. Verfahren zur Herstellung eines bromierten Bisphenols in hoher Ausbeute und hoher Reinheit, wobei das Verfahren die Schritte umfaßt, daß man
(A) einen ersten Strom, der in Methanol gelöstes Brom umfaßt, einem Aufprallmischer zuführt;
(B) einen zweiten, das in Methanol gelöste Bisphenol umfassenden Strom und - nach wenigstens einem Zyklus des Verfahrens - bromiertes Bisphenol mit dem ersten Strom in dem Aufprallmischer aufprallen läßt, um die Ströme schnell zu vermischen und eine Mischung daraus zu bilden;
(C) die Mischung einem Reaktionsgefäß zuführt, das Reaklorinhaltsstoffe enthält, die das in Methanol gelöste Bisphenol und - nach wenigstens einem Zyklus des Verfahrens - das bromierte Bisphenol umfassen;
(D) einen Teil der Reaktorinhaltsstoffe aus dem Reaktionsgefäß entfernt, um den zweiten Strom zu bilden; und
(E) Schritte (A), (B), (C) und (D) fortführt, bis die gewünschte Menge an Brom zugeführt wurde.

2. Verfahren nach Anspruch 1, worin das Bisphenol Bisphenol A ist und das bromierte Bisphenol Tetrabrombisphenol A ist.

3. Verfahren nach Anspruch 2, worin bei Start des Verfahrens das Gewichtsverhältnis des Methanpols zu dem Bisphenol A in dem zweiten Strom 1 bis 6 : 1 und das Gewichtsverhältnis des Broms und Methanols in dem ersten Strom 1 bis 3 : 1 beträgt.

4. Verfahren nach Anspruch 3, worin bei Start des Verfahrens das Gewichtsverhältnis des Methanols zu dem Bisphenol A in dem zweiten Strom 1,5 bis 3 : 1 beträgt und die Menge an zugeführtem Brom 4,0 bis 4,1 Mol Brom pro Mol Bisphenol A, das in dem Verfahren eingesetzt wird, beträgt.

5. Verfahren nach Anspruch 4, worin das Volumenverhältnis des zweiten Stroms zu dem ersten Strom innerhalb des Bereichs von 10 bis 20 : 1 liegt.

6. Verfahren nach irgendeinem der Ansprüche 1 bis 5, worin die Temperatur in dem Reaktionsgefäß während des Verlaufs der Reaktion Rückflußbedingungen erreicht.

7. Verfahren nach irgendeinem der Ansprüche 1 is 6, worin das bromierte Bisphenol aus dem Verfahren gewonnen wird.

8. Verfahren nach Anspruch 7, worin der Schritt des Gewinnens durch Schritte bewirkt wird, die das schnelle Abkühlen (quenchen) der Reaktorinhaltsstoffe mit Wasser unter Fällung des bromierten Bisphenols und Abfiltrieren des gefällten bromierten Bisphenols von den Reaktorinhaltsstoffen umfaßt.

9. Vorrichtung, die zur Herstellung einer bromorganischen Verbindung in hoher Ausbeute und hoher Reinheit geeignet ist, wobei die Vorrichtung umfaßt: ein Reaktionsgefäß mit einem Auslaß am Boden und einem Einlaß, eine Auslaßleitung, die eine Fließverbindung von dem Auslaß am Boden der Vorrichtung zum Ansaugstutzen einer Pumpe bildet, eine Steigleitung, die eine Fließverbindung von der Ablaßöffnung der Pumpe zum Seiteneinlaß eines ringförmigen Aufprallmischers bildet, eine Bromquelle und eine Lösungmittelquelle, die operativ mittels einer Brom-Lösungsmittel-Leitung mit dem Endeinlaß des ringförmigen Aufprallmischers verbunden ist, wobei der ringförmige Aufprallmischer ein äußeres, im wesentlichen zylindrisches Gehäuse, ein im wesentlichen zylindrisches hohles Verteilerbauteil, das im wesentlichen an einem Ende geschlossen ist und axial im Innern des zylindrischen Gehäuses und von diesem räumlich entfernt angeordnet ist und abdichtbar mit dem Gehäuse an einem Ende des Gehäuses verbunden ist und einen ringförmigen Raum zwischen dem Verteilerbauteil und dem Gehäuse bildet, Öffnungen in der Seitenwandung des Verteilerbauteils, die eine Fließverbindung vom Innenraum des Verteilerbauteils in den ringförmigen Raum bilden, einen Endeinlaß in das Verteilerbauteil, der gegenüber dem geschlossenen Ende angeordnet ist, wobei der Endeinlaß operativ mit der Brom-Lösungsmittel-Leitung verbunden ist, umfaßt, ein äußeres axiales zylindrisches Bauteil, das um das zylindrische Gehäuse herum und räumlich von diesem entfernt angeordnet ist, wo bei beiden Enden des axialen zylindrischen Bauteils abdichtbar mit dem zylindrischen Gehäuse verbunden sind und eine ringförmige Kammer bilden, Flüssigkeitsdurchlauf-Einrichtungen entlang der Peripherie des zylindrischen Gehäuses, die einen Durchlauf von der ringförmigen Kammer in den ringförmigen Raum bilden, einen Einlaß in das zylindrische Bauteil, der als Seiteneinlaß des ringförmigen Aufprallmischers fungiert, und eine Zufuhrleitung, die eine Fließverbindung von einem Auslaß in dem zylindrischen Gehäuse, der entfernt von dem Verteilerbauteil angeordnet ist, zu dem Einlaß des Reaktionsgefäßes bildet.

10. Vorrichtung nach Anspruch 9, worin die Lösungsmittelquelle eine Methanolquelle ist.

11. Vorrichtung nach Anspruch 10, welche weiter dadurch gekennzeichnet ist, daß sie eine statische Mischvorrichtung in der Brom-Lösungsmittel-Leitung aufweist.

## Revendications

1. Procédé de préparation d'un bisphénol bromé en un haut rendement et une grande pureté, ledit procédé comprenant :
(A) l'introduction d'un premier courant comprenant du brome dissous dans du méthanol dans un mélangeur par collision ;
(B) le contact par collision d'un second courant comprenant le bisphénol dissous dans du méthanol et, après au moins un cycle dudit procédé, du bisphénol bromé avec ledit premier courant dans ledit mélangeur par collision pour le mélange rapide desdits courants et pour la formation d'un mélange de ces courants ;
(C) l'envoi dudit mélange à un récipient réactionnel renfermant le contenu du réacteur qui comprend le bisphénol dissous dans du méthanol et, après au moins un cycle dudit procédé, le bisphénol bromé ;
(D) le prélèvement d'une portion du contenu du réacteur dudit récipient réactionnel pour former ledit second courant ; et
(E) le maintien de la mise en oeuvre des étapes (A), (B), (C) et (D) jusqu'à ce que la quantité désirée de brome ait été introduite.

2. Procédé suivant la revendication 1, dans lequel le bisphénol est le bisphénol-A et le bisphénol bromé est le tétrabromobisphénol-A.

3. Procédé suivant la revendication 2, dans lequel, au début de la mise en oeuvre du procédé, le rapport pondéral du méthanol au bisphénol-A dans le second courant est égal à 1-6:1 et le rapport pondéral du brome au méthanol dans le premier courant est égal à 1-3:1.

4. Procédé suivant la revendication 3, dans lequel, au début de la mise en oeuvre du procédé, le rapport pondéral du méthanol au bisphénol-A dans le second courant est égal à 1,5-3:1 et la quantité de brome introduite est égale à 4,0-4,1 moles de brome par mole de bisphénol-A utilisée dans ledit procédé.

5. Procédé suivant la revendication 4, dans lequel le rapport, en volume, du second courant au premier courant est compris dans l'intervalle de 10:1 à 20:1.

6. Procédé suivant l'une quelconque des revendications 1 à 5, dans lequel la température dans le récipient réactionnel atteint les conditions de reflux au cours de la réaction.

7. Procédé suivant l'une quelconque des revendications 1 à 6, dans lequel le bisphénol bromé est séparé après mise en oeuvre dudit procédé.

8. Procédé suivant la revendication 7, dans lequel la séparation est effectuée par les étapes comprenant la désactivation du contenu du réacteur avec de l'eau pour précipiter le bisphénol bromé et la filtration dudit bisphénol bromé précipité pour le séparer du contenu du réacteur.

9. Appareil apte à la préparation d'un composé organique bromé en un haut rendement et une grande pureté, ledit appareil comprenant un récipient réactionnel ayant un orifice inférieur de sortie et un orifice d'admission, un conduit de sortie formant un trajet d'écoulement à partir dudit orifice inférieur de sortie jusqu'à l'entrée d'une pompe, un conduit montant formant un trajet d'écoulement de l'orifice de déchargement de ladite pompe à l'orifice latéral d'admission d'un mélangeur annulaire par collision, une source de brome et une source de solvant connectées de manière fonctionnelle par un conduite de brome-solvant à l'orifice terminal d'admission dudit mélangeur annulaire par collision, ledit mélangeur annulaire par collision comprenant une enveloppe extérieure pratiquement cylindrique, un élément distributeur creux pratiquement cylindrique, pratiquement clos à une extrémité et situé axialement à l'intérieur, et espacé, de ladite enveloppe cylindrique et connecté de manière étanche avec ladite enveloppe à une extrémité de ladite enveloppe formant un espace annulaire entre ledit élément de distribution et ladite enveloppe, des orifices dans la paroi latérale dudit élément de distribution formant un trajet d'écoulement de l'intérieur dudit élément de distribution dans ledit espace annulaire, un orifice terminal d'admission dans ledit élément de distribution opposé à ladite extrémité close, ledit orifice terminal d'admission étant connecté de manière fonctionnelle audit conduit de brome-solvant, un élément cylindrique axial extérieur autour et espacé de ladite enveloppe cylindrique, les deux extrémités dudit élément cylindrique axial étant connectées de manière étanche à ladite enveloppe cylindrique formant une chambre annulaire, des moyens de passage de fluide autour de la circonférence de ladite enveloppe cylindrique formant un passage de ladite chambre annulaire dans ledit espace annulaire, un orifice d'admission dans ledit élément cylindrique fonctionnant comme ledit orifice latéral d'admission dudit mélangeur annulaire par collision, un conduit d'alimentation formant un trajet d'écoulement d'un orifice de sortie dans ladite enveloppe cylindrique loin dudit élément de distribution audit orifice d'admission dudit récipient réactionnel.

10. Appareil suivant la revendication 9, dans lequel la source de solvant est une source de méthanol.

11. Appareil suivant la revendication 10, caractérisé en outre par la possession de moyens de mélange statiques dans le conduit de brome-solvant.
